# EUROPEAN PATENT SPECIFICATION

(11) **EP 0 898 478 B1**
(45) Date of publication and mention of the grant of the patent: **01.02.2006**
(21) Application number: 97920907.9
(22) Date of filing: 05.05.1997
(51) Int. Cl.: A61K 38/21

(54) **STIMULATION OF HOST DEFENCE MECHANISMS AGAINST TUMORS**
STIMULIERUNG VON WIRTSEIGENEN ABWEHRMECHANISMEN GEGEN TUMORE
STIMULATION DES MECANISMES DE DEFENSE D'HOTES CONTRE LES TUMEURS

(30) Priority: 09.05.1996 AU PN976596
(43) Date of publication of application: 03.03.1999
(73) Proprietor: Pharma Pacific Pty. Ltd., Brighton-Le-Sands, NSW 2216 (AU)
(72) Inventor: TOVEY, Michael, Gerard, F-75005 Paris (FR); KAIDO, Thomas, James, San Diego, CA 92109 (US)
(74) Representative: Walton, Seán Malcolm
(86) International application number: PCT/IB1997/000594
(87) International publication number: WO 1997/041886

(56) References cited:
- EP-A- 0 396 903
- WO-A-82/00588
- WO-A-92/10207
- WO-A-93/21229
- WO-A-97/41884
- AU-A- 1 222 788
- US-A- 4 605 555
- US-A- 4 820 515
- US-A- 5 286 748
- THE JOURNAL OF INFECTIOUS DISEASES, Volume 150, No. 2, August 1984, TOBIAS C.S. et al., "Intranasally Applied Recombinant Leukocyte A Interferon in Normal Volunteers. II. Determination of Minimal Effective and Tolerable Dose".
- THE JOURNAL OF INFECTIOUS DISEASES, Volume 156, No. 1, July 1987, HAYDEN F.G. et al., "Human Nasal Mucosal Responses to Topically Applied Recombinant Leukocyte A Interferon".
- THE JOURNAL OF INFECTIOUS DISEASES, Volume 148, No. 3, September 1983, SAMO T.C. et al., "Efficacy and Tolerance of Intranasally Applied Recombinant Leukocyte A Interferon in Normal Volunteers".
- THE JOURNAL OF INFECTIOUS DISEASES, Volume 148, No. 5, November 1983, HAYDEN F.G. et al., "Human Tolerance and Histopathologic Effects of Long-Term Administration of Intranasal Interferon-alpha2".
- JOURNAL OF INTERFERON AND CYTOKINE RESEARCH, VOL 19:853-857, 1999
- VETERINARY IMMUNOLOGY AND IMMUNOPATHOLOGY, 49: 347-358, 01.1996
- KAIDO T.J.: "Intranasal administartion of IFN-alpha/beta Inhibits the development of visceral tumor metastases" JOURNAL OF INTERFERON AND CYTOKINE RESEARCH, vol. 17, 1997, pages 31-36,
- DATABASE MEDLINE US NATIONAL LIBRARY OF MEDICINE (NLM), BETHESDA, MD, US; Database accession no. NLM6381610 & HAYDEN F G; GWALTNEY J M JR: "INTRANASAL INTERFERON-ALPHA-2 TREATMENT OF EXPERIMENTAL RHINOVIRAL COLDS" JOURNAL OF INFECTIOUS DISEASES, vol. 150, no. 2, 1984, pages 174-180,

## Description

This invention relates to methods of stimulation of host defense mechanisms against pathological conditions in a host mammal by administration of interferon via the oromucosa. In particular, the invention is applicable to methods of treatment of cancer.

### BACKGROUND OF THE INVENTION

Alpha interferons are used widely for the treatment of a variety of hematological malignancies including hairy cell leukemia, chronic myelogenous leukemia, low grade lymphomas, cutaneous T-cell lymphomas; and solid tumors such as renal cell carcinoma, melanoma, carcinoid tumors and AIDS-related Kaposi's sarcoma (Gutterman, J.U., *Proc. Natl. Acad Sci. USA,* 1994 **91**: 1198-1205; WO 8803411). Antitumor effects are usually seen at high dosage levels, often of the order of tens of millions of units of interferon-α (IFN-α), administered by parenteral injection. Interferon-α is a Type I interferon, a class which also includes the β and ω interferons. Although a number of routes of administration, including intravenous, subcutaneous, intramuscular, topical, and intralesional injection, are commonly employed for the administration of type I interferons, the oral route has not been generally used, because interferons are proteins which are considered to be inactivated by proteolytic enzymes and which are not absorbed appreciably in their native form in the gastrointestinal tract. Indeed a number of studies have failed to detect interferons in the blood following oral administration (Cantell and Pyhäla, *J*. *Gen. Virol.,* 1973 **20**: 97-104; Wills *et al, J. IFN Res*., 1984 **4**: 399-409; Gilson *et al, J. IFN Res*., 1985 **5**: 403-408).

There have been a number of anecdotal reports of efficacy of low doses of interferon administered as a nasal spray or as an oral liquid formulation in the treatment of a variety of conditions, particularly influenza. A series of patent specifications has described the use of low doses of orally administered interferon of heterologous species origin for the treatment of infectious rhinotracheitis ("shipping fever") in cattle, and of feline leukemia, and also treatment of other conditions, for enhancement of efficiency of vaccines; for improving the efficiency of food utilization; and for prevention of bovine theileriosis. See U.S. Patent No. 4,462,985, Australian Patent No. 608519, Australian Patent No. 583332 and U.S. Patent No. 5,215,741 respectively. In addition U.S. Patent No. 5,017,371 discloses the use of interferon in this way for treatment of side-effects of cancer chemotherapy or radiotherapy. In these specifications, the interferon used was human interferon-α prepared by the method of Cantell, administered in phosphate buffered saline, at a dose of 0.41 to 5 IU per pound body weight. While these specifications suggest that such low doses of interferon administered to the oropharyngeal mucosa, preferably in a form adapted for prolonged contact with the oral mucosa, may be efficacious for treatment of a wide variety of conditions including cancer, the experimental evidence for conditions other than shipping fever, feline leukemia, canine parvovirus and theileriosis is largely anecdotal. In particular, no properly controlled trials of this treatment in any animal model for human cancers are presented.

In contrast, the invention disclosed herein is based upon the first controlled study in an animal model of the efficacy of oromucosally administered interferon for the treatment of cancer.

### SUMMARY OF THE INVENTION

This invention provides a method for treating cancer in a human via administering to the human a therapeutically effective amount of an interferon via oromucosal contact. The amount of interferon administered is less than an amount which induces a pathological response when administered parenterally. In particular, the invention provides a method for treating multiple myeloma, hairy cell leukemia, chronic myelogenous leukemia, low grade lymphoma, cutaneous T-cell lymphoma, carcinoid tumors, cervical cancer, sarcomas including Kaposi's sarcoma, kidney tumors, carcinomas including renal cell carcinoma, hepatic cellular carcinoma, nasopharyngeal carcinoma, hematological malignancies, colorectal cancer, glioblastoma, laryngeal papillomas, lung cancer, colon cancer, malignant melanoma, and brain tumors including malignant brain tumors. In one embodiment, the method is generally applicable in the treatment of tumors of non-viral etiology.

The oromucosal administration may involve administering an effective dose of interferon in a single dose or the effective dose may be administered in a plurality of smaller doses over a period of time sufficient to elicit host defense stimulation equivalent to that of a single dose. Likewise, the effective dose of interferon may be administered continuously over a period of time sufficient to elicit host defense stimulation equivalent to that of a single dose.

The method is practiced by administering from about 1 X 10⁴ IU to about 20X10⁶ IU of interferon, most preferably from about 1 X 10⁴ to about 1 X 10⁶ IU of interferon, provided that the chosen dose is one which does not induce a parenteral pathological response, as defined herein, or is less than the amount which induces a pathological response when administered parenterally. These dose ranges generally refer to homologous interferon α in man. For another type of interferon the dose that will induce a pathological response may differ from that induced by homologous interferon α in man. A physician treating a patient with a particular type of interferon will be able to readily identify the suitable dose range for the patient to be treated.

A pharmaceutical composition for oromucosal administration may comprise a therapeutically effective amount of at least one interferon. The composition may be provided as a solution, tablet, lozenge, gel, syrup, paste, or controlled release oromucosal delivery system. Optionally, the composition may contain buffers, stabilizers, thickening agents, absorption, and viscosity enhancers, and the like.

The pharmaceutical composition may be provided in unit dosage form having from 1 X 10⁴ IU to 20X10⁶ IU of interferon, most preferably from 1 X 10⁴ to 1 X 10⁶ IU of interferon.

The method may be practiced either as the sole therapeutic approach, or as an adjunct to chemo or radiation therapy, or with other cytokines, such as interleukin-2, 12, or 15, or with IFN-inducers.

The method is conducted using a Type I or II interferon, selected from α, β, γ, ω, and consensus interferons, most preferably with a recombinant IFN-α.

### DETAILED DESCRIPTION OF THE INVENTION

The invention will now be described in detail by way of reference only using the following definitions and examples. All patents and publications referred to herein are expressly incorporated by reference.

### Definitions

As used herein, "interferon" refers to a Type I or Type II interferon, including those commonly designated as α, β, γ, and ω, and mixtures thereof, including the consensus sequence. Interferons are available from a wide variety of commercial sources and are approved for the treatment of numerous indications. The interferon may be from natural sources, but is preferably a recombinant product. For the purposes of the invention, the term "interferon" also includes polypeptide fragments which have interferon activity, and chimeric or mutant forms of interferon in which sequence modifications have been introduced, for example to enhance stability, without affecting the nature of their biological activity, such as disclosed in U.S. Patent Nos. 5,582,824, 5,593,667, and 5,594,107 among others.

Optionally the interferon may be administered concurrently with an inducer of interferon synthesis and release. The inducer may be administered together with the interferon, or may be administered separately. Inducers of interferon include, for example, polynucleotides such as poly I:C; preferably a low molecular weight, orally administrable interferon inducer is used. Suitable inducers are known in the art, for example, Tilorone (U.S. Patent No 3592819; Albrecht *et al*, *J*. *Med. Chem.* 1974 **17**: 1150-1156) and the quinolone derivative Imiquimod (*Savage et al*; *Brit. J Cancer,* 1996 **74**: 1482-1486).

The methods and compositions of the invention may optionally be used in conjunction with one or more other treatments for the specific condition, and the attending physician or veterinarian will readily be able to select such other treatment as may be appropriate in the circumstances.

In one embodiment, the invention provides a method of treatment of a cancer in a human, comprising the step of administering interferon as described above. The cancer may be metastatic cancer.

While the method of the invention may be used without concurrent treatment with other agents, it is contemplated that this embodiment of the invention will be particularly useful in the following settings:
a) as adjuvant therapy, subsequent to surgery, chemotherapy, or radiotherapy given by standard protocols;
b) for treatment of interferon-sensitive cancers, the method of the invention is utilized either alone or in conjunction with conventional chemotherapy or radiotherapy; and
c) for treatment of interferon-resistant cancers, the method of the invention is utilized either alone or most preferably in conjunction with conventional chemotherapy or radiotherapy.

The above methods are directed at inducing and/or maintaining remission of disease. By "in conjunction with other treatment" is meant that the interferon is administered before, during and/or after the radiotherapy or other chemotherapy. The most suitable protocol will depend on a variety of factors, as discussed below.

In particular, it is contemplated that the method of the invention will preferably be used in conjunction with at least one other treatment selected from the group consisting of chemotherapy using cytostatic drugs, one or more other cytokines which have anti-cancer activity but which have a different mechanism of action from that of interferon, anti-angiogenic agents, and agents which potentiate the activity of interferon. Preferably the second cytokine is interleukin-1 (IL-1), interleukin-2 (IL-2) interleukin-12 (IL-12), or interleukin-15 (IL-15); preferably the angiogenesis inhibitor is AGM-1470 [(Chloroacetyl)-carbamic acid (3R-(3α, 4α (2R*, 3R*), 5β, 6β))-5-methoxy-4-(2-methyl-3-(3-methoxy-2-butenyl)oxiranyl)-1-oxaspiro(2.5)oct-6-yl ester]; preferably the interferon-potentiating treatment is hyperthermia or arginine butyrate.

Preferred cytostatic drugs to be administered in conjunction with interferon include but are not limited to cyclophosphamide, cisplatin, carboplatin, carmustine (BCNU; N,N-Bis(2-chloroethyl)-N-nitrosourea), methotrexate, adriamycin, α-difluoromethylomithine, and 5-fluorouracil.

The cancers susceptible to this method include but are not limited to cancers which respond to parenteral administration of high doses of IFN-α, such as hematological malignancies, *e.g.* multiple myeloma, hairy cell leukemia, or chronic myelogenous leukemia, low grade lymphomas, cutaneous T cell lymphoma, solid tumors such as renal cell carcinoma and melanoma, carcinoid tumors, or AIDS-associated Kaposi's sarcoma, in particular tumors of non-viral etiology.

In the preparation of the pharmaceutical compositions used in this invention, a variety of vehicles and excipients for IFN may be used, as will be apparent to the skilled artisan. Representative formulation technology is taught in, *inter alia,* Remington: The Science and Practice of Pharmacy, 19th ed., Mack Publishing Co., Easton, PA, 1995, and its predecessor editions. The IFN formulation may comprise stability enhancers, such as glycine or alanine, as described in U.S. Patent No. 4,496,537, and/or one or more carriers, such as a carrier protein. For example, for treatment of humans pharmaceutical grade human serum albumin, optionally together with phosphate-buffered saline as diluent, is commonly used. Where the excipient for IFN is human serum albumin, the human serum albumin may be derived from human serum, or may be of recombinant origin. Normally when serum albumin is used it will be of homologous origin.

The IFN may be administered by any means which provides contact of the IFN with the oromucosal cavity of the recipient. Thus it will be clearly understood that the invention is not limited to any particular type of formulation. The present specification describes administration of IFN deep into the oromucosal cavity; this may be achieved with liquids, solids, or aerosols, as well as nasal drops or sprays. Thus the invention includes, but is not limited to, liquid, spray, syrup, lozenges, buccal tablets, and nebuliser formulations. A person skilled in the art will recognize that for aerosol or nebuliser formulations the particle size of the preparation may be important, and will be aware of suitable methods by which particle size may be modified.

In one aspect, the interferon is administered in a single daily dose. Alternatively, the interferon is administered in a plurality of lower doses, distributed over time, so that the net effect is equivalent to the administration of the single higher dose. One approach to this delivery mode is via the provision of a sustained or controlled release device adhered to or implanted in the oromucosal cavity and designed to release interferon over time in an amount equivalent to a single high dose.

Representative formulations of interferon for oromucosal use include the following (all % are w/w):

Tablet: Dextrose BP 45 %; gelatin BP 30 %; wheat starch BP 11 %; carmellose sodium BP 5 %; egg albumin BPC 4 %; leucine USP 3 %; propylene glycol BP 2%; and 1 X 10⁶ IU IFN-α2. The tablet may be used as is and allowed to slowly dissolve in the mouth or may be dissolved in water and held in the mouth in contact with the oromucosa as needed.

An interferon paste may be prepared, as described in U.S. Patent No. 4,675,184, from glycerin 45%, sodium CMC 2%, citrate buffer (pH 4.5) 25%, distilled water to 100%, and 1 X 10⁶ IU IFN.-α2 The interferon paste may be adhered to the buccal mucosa.

Likewise, a gargle or a syrup may be prepared by adding the desired amount of interferon to a commercially available mouthwash or cough syrup formulation.

Within the specific dose ranges referred to above, the optimal treatment in any individual case will depend on the nature of the condition concerned, the stage of disease, previous therapy, other continuing therapy, the general state of health of the patient, the sensitivity of the subject to interferon, *etc*., and therefore will be at the physician's discretion, bearing in mind all these circumstances. The length of treatment will of course vary with the condition being treated, for example, treatment of a slow-growing cancer, such as prostate cancer, would be expected to involve a different course of treatment than treatment of a rapidly growing cancer, such as hepatic cellular carcinoma.

The effective dose disclosed herein is one which does not generate a pathological response in the patient when administered parenterally. A pathological response may be acute, chronic, or cumulative, and may be manifested by changes in blood chemistry, such as leukopenia, bone marrow depression, or other histological parameters. As used herein, a pathological response includes adverse side effects, such as fever, malaise, or flu-like symptoms, vascular reactions, such as phlebitis, and local inflammatory reactions at the site of injection. Such responses will vary considerably among the patient population in view of individual variations in sensitivity to interferon. A simple test for identifying an acceptable low dose of interferon for oromucosal therapy is to inject the patient with the putative acceptable dose, based upon considerations of age, weight, indication, progression, etc. and ascertain if the injection produces a pathological response as defined herein, with local irritation at the site of injection being the most readily ascertainable criterion. If no adverse response is noted, then the same dose may be administered oromucosally. If there is an undesirable response, then the process is repeated at a lower dose, until a non-pathological dose is identified.

For many patients, it is expected that oromucosal doses will be approximately the same as those known to be well tolerated and effective in existing approved parenteral protocols. Therefore, for purposes of specificity, an acceptable low dose of interferon may be from about 1 X 10⁴ IU to about 20 X 10⁶ IU of interferon per day, most preferably from about 1 × 10⁴ IU to about 1 X 10⁶ IU of interferon per day, provided that the dose is one which does not induce a pathological response when administered parenterally. In one embodiment, the total dose may be administered in multiple lower doses over time, or even may be delivered continuously or in a pulsatile manner from a controlled release device adhered to or implanted in the oromucosa.

### INTERFERONS AND INTERFERON FORMULATIONS

### Mouse IFN-α/β

Mouse IFN-α/β (Mu IFN-α/β) was prepared from cultures of C243-3 cells induced with Newcastle disease virus (NDV) and purified as described previously (Tovey *et al, Proc. Soc. Exp. Biol. and Med*., 1974 **146**: 809-815). The preparation used in this study had a titer of 4 x10⁶ International Units (IU)/ml and a specific activity of 5 x 10⁷ IU/mg protein as assayed on mouse 929 cells challenged with vesicular stomatitis virus (VSV) as described previously (Tovey *et al, Proc. Soc. Exp. Biol. and Med.,* 1974 **146**: 809-815). The preparation was standardized against the international reference preparation of murine IFN-α/β of the National Institutes of Health (NIH) (G-002-9004-541).

### Human IFN-α-1-8

Recombinant human IFN-α 1-8 (Hu IFN-α 1-8; BDBB lot no. CGP 35269-1, Ciba-Geigy, Basel, Switzerland) was prepared and purified as described previously (Meister *et al, J. Gen. Virol*., 1986 **67**: 1633-1643). The preparation used in this study had a titer of 70 x 10⁶ IU/ml on homologous human WISH cells challenged with VSV as described previously (Tovey *et al*, *Nature,* 1977 **267**: 455-457), and a titer on heterologous mouse L929 cells of 1 x 10⁶ IU/ml. The preparation was standardized against both the NIH human IFN-α international reference preparation (G-023-901-527) and the NIH murine IFN-α/β standard (G-002-9004-5411). The specific activity of the IFN preparation was 2 x 10⁸ IU/mg protein.

### RECOMBINANT MURINE INTERFERON-α

Recombinant murine interferon-α was purchased from Life Technologies Inc. The preparation used in this study (lot no. HKK404) had a titer of 6 x 10⁶ IU/ml and a specific activity of 6 x 10⁸ IU/mg protein as assayed on mouse L929 cells challenged with VSV (Tovey et al, Proc. Soc. Exp. Biol. Med., 1974, 146:406-415).

### RECOMBINANT MURINE INTERFERON β

Recombinant murine interferon β was purchased from R & D Systems Inc. The preparation used in this study (lot no. 1976-01S) had a titer of 3.2 X 10⁴ IU/ml and a specific activity of 8 X 10⁶ IU/mg protein as assayed on mouse L929 cells challenged with VSV (Tovey et al, proc. Soc. Exp. Biol. Med., 1974, 146:406-415).

### RECOMBINANT MURINE INTERFERON γ

Recombinant murine interferon γ was purchased from R & D Systems Inc. The preparation used in this study (2580-03SA) had a titer of 2 X 10⁵ IV/ml and a specific activity of 1 X 10⁷ IU/mg protein as assayed on mouse L929 cells challenged with VSV (Tovey et al, Proc. Soc. Exp. Biol. Med., 1974, 146:406-415).

All the interferon preparations were titrated simultaneously in the same assay and standardized against the international reference preparation of murine interferon α/β of the US National Institutes of Health (G-002-9004-5411).

Both murine interferon α/β and recombinant murine interferons were taken up in Ferimmune™ excipient prior to administration.

### EXCIPIENT

Interferon preparations were diluted in phosphate buffered saline (PBS) containing bovine serum albumin (BSA). Bovine serum albumin fraction V (RIA grade; immunoglobulin free; Cat. no. A7888; Sigma; USA) was dissolved at a final concentration of 100 µg/ml in PBS (pH 7.4) and sterilized by filtration (0.2 µ, Millex-GV, Millipore, USA).

In the experiments described herein the interferon preparations were diluted in a proprietary excipient. The excipient used was as follows, supplied in the form of tablets (Ferimmune™, Pharma Pacific):

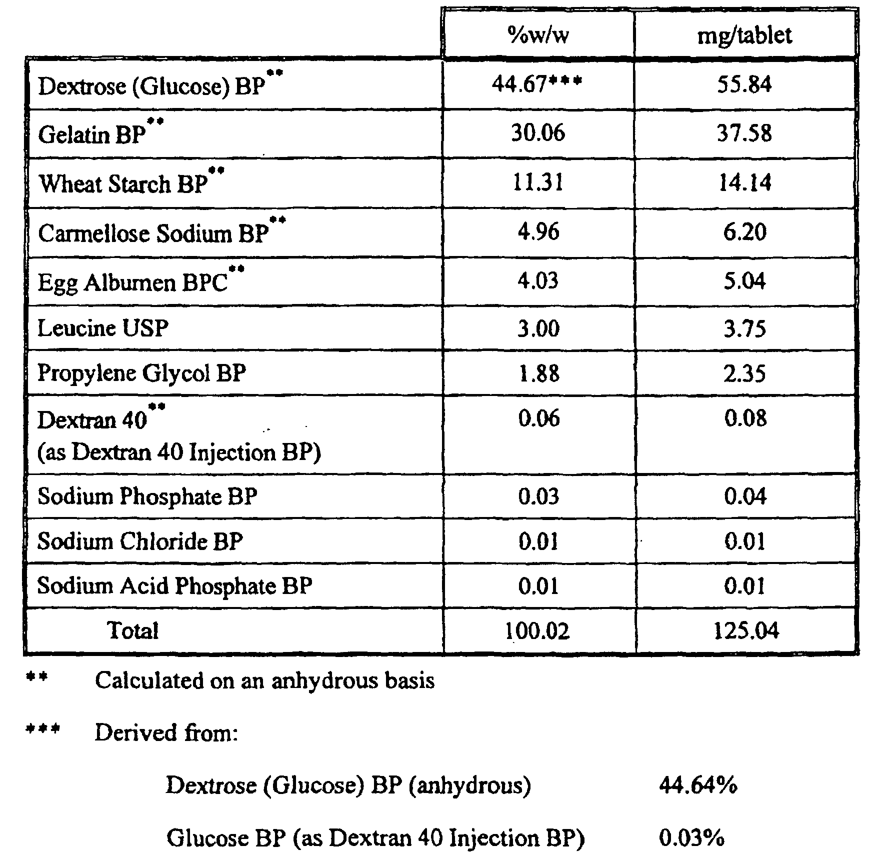

A single tablet was dissolved in 1.5 ml phosphate buffered saline, centrifuged at 16,000 g for 15 m, and then sterile filtered (0.2 µ, Millex-GV, Millipore, USA), and stored at 4°C prior to use. Excipient was prepared daily prior to use.

### INTERFERON DELIVERY SYSTEM

Preliminary experiments showed that the application of 5 µl of crystal violet to each nostril of a normal adult mouse using a P20 Eppendorf micropipette resulted in an almost immediate distribution of the dye over the whole surface of the oropharyngeal cavity. Staining of the oropharyngeal cavity was still apparent some 30 minutes after application of the dye. Essentially similar results were obtained using ¹²⁵I-labeled recombinant human IFN-α 1-8 applied in the same manner. This method of administration was therefore used in all subsequent experiments.

For the purposes of the animal experiments described in this specification, it will be clearly understood that the expressions "oromucosal" or "oropharyngeal" or "intranasal/oral" or "intranasal plus oral" or "in/or" with reference to the route of administration of IFN is to be taken to mean administration of the IFN preparation deep into the nasal cavity so that it is rapidly distributed into the oromucosal cavity, *i*.*e*. the mouth and throat of the recipient mammal, so as to make contact with the mucosa lining this cavity.

### FRIEND ERYTHROLEUKAEMIA CELLS

The IFN-α/β resistant clone, 3C18, of Friend erythroleukaemia cells (FLC) was obtained from Dr E. Affabris, Rome and is described in detail by Affabris *et al*, 1982 (*Virology,* **120:** 441-452). These cells were subsequently maintained by *in vivo* passage. Briefly, DBA/2 mice were inoculated by intraperitoneal injection (ip) with approximately 100 LD₅₀ of 3C18 cells and one week later the tumor cells were harvested from the peritoneum of the mice, counted and other mice were again inoculated with 100 LD₅₀ of 3C18 cells. This procedure was repeated for 60 to 100 passages. It has been shown that the 3C18 cells used at the 60th to 100th *in vivo* passage are highly metastatic for the liver and spleen (Gresser *et al, Int*. *J*. *Cancer,* 1987 **39**: 789-792). The phenotype of IFN resistance was confirmed routinely by cultivating the *in vivo* passaged cells *in vitro* in the presence of IFN-α/β (Belardelli *et al*, *Int*. *J*. *Cancer,* 1982 **30**: 813-820).

The interferon compositions of the present invention are also active against the L1210R6 clone of L1210 lymphoma cells isolated in our laboratory (Gresser et al., 1974, Interferon and cell division. IX. Interferon-resistant L1210 cells : Characteristics and Origin. J. Nat. Cancer Inst., 52:553-559) and EL4 transplantable tumor derived from mice inoculated with the chemical carcinogen 1-2 dimethyl benzanthrein (Gorer, P.A., 1950, Br. J. Cancer, 4:372-381).

### ANIMALS

The mice used in this study were obtained from a specific pathogen-free colony (IFFA CREDO, France). They were housed in a specific pathogen-free animal facility at the Institut Federatif CNRS at Villejuif according to EEC standards.

### INTERFERON BIOASSAY

Interferon was assayed according to a conventional method. Briefly, samples (20 µl) were diluted in 80 µl of Eagle's Minimal Essential Medium (MEM) (Gibco, France) containing 2% heat-inactivated Fetal Calf Serum (FCS) (Gibco, France) and added to each well of a microtitre plate (Falcon, cat. no. 3072) using a multichannel micro-pipette (Finnpipette, Labsystem, 50-300 µl). WISH or L929 cells (2 x 10⁴ cells/well) were added in 100 µl of MEM containing 2% FCS and incubated overnight at 37°C in an atmosphere of 5% CO₂ in air (Forma 3029 CO₂ incubator). The cells were then examined for any signs of toxicity using an Olympus IM GLDW inverted microscope equipped with a 10X objective. Samples which did not exhibit detectable toxicity were then subjected to serial two-fold dilutions starting from an initial 1:10 dilution in a total volume of 200 µl of Eagle's MEM containing 2% FCS, by carrying forward 100 µl of diluted material with a multichannel micropipette, in a microplate containing 100 µl per well of fresh Eagle's MEM containing 2% FCS, Appropriate serial two-fold dilutions of the NIH human IFN-α reference standard (G-023-901-527) or the NIH Mu IFN-α/β reference standard (G-002-9004-5411) were also prepared. WISH or L929 cells (2 x 10⁴ cells/well) in 100 µl of Eagle's MEM containing 2% FCS were then added to each plate where appropriate and incubated overnight at 37°C in an atmosphere of 5% CO₂ in air. The cell monolayers were then checked for any signs of toxicity and in the absence of any apparent toxicity, the culture was aspirated and replaced with 200 µl of Eagle's MEM containing 2% FCS containing 100 TCID₅₀ of VSV (2 x 10⁻⁴ VSV₂₃ for WISH cells, or 10⁻⁵ VSV₂₃ for L929 cells). The plates were then incubated overnight at 37°C in an atmosphere of 5% CO₂ in air. The cell monolayers were then examined for specific viral cytopathic effect using an Olympus IM ULWD inverted microscope. Interferon titers were determined from the reciprocal of the dilution which gave 50% protection against specific viral cytopathic effect, and are expressed in international reference units/ml (IU/ml).

### Example 1 Anti-Tumor Activity of Oromucosally Administered Interferon-α in Mice Challenged with Friend Erythroleukaemia Cells

In order to establish whether IFN-α administered by the intranasal/oral (in/or) route increases the survival of mice challenged with highly metastatic FLC cells, groups of six 7-8 week-old male DBA/2 mice were challenged intravenously (iv) with 1 x 10⁵ FLC on day 0.

Each group of mice was treated twice a day for 10 consecutive days by the in/or route with either 10⁴ IU of a natural mixture of multiple murine IFN-α subtypes (Mu IFN-α) in 10 µl BSA-PBS, or with an equal volume of a mock IFN preparation, which was produced and purified in the same manner as the IFN preparation with the exception of the omission of the virus inducer. The mock IFN preparation did not exhibit detectable IFN activity when assayed in parallel with the purified Mu IFN-α preparation. The results are shown in Table 1.

**Table 1**

| Group | Treatment | Day of Death | Mean day of death ±SE |
|---|---|---|---|
| 1 | Mock Mu IFN-α | 9,9,10,10,10,10 | 9.6 ±0.2 |
| 2 | Mu IFN-α 10⁴ IU | 28,31,36>100>100 | 31.7 ± 2.3** |

| | | | |
|---|---|---|---|
| ** Calculated for dead animals only | | | |

Oromucosal administration of 10⁴ IU of Mu IFN-α twice a day dramatically increased the survival time of mice injected with FLC. In fact 2 of 5 mice were effectively cured as they were alive and well 100 days after challenge with 2 x 10⁴ LD₅₀ of FLC, despite cessation of IFN-α treatment after only 20 days. The results of controlled tests on groups of 10 adult DBA/2 mice infected with 2 X 10⁴ LD₅₀ of FLC and treated oromucosally with 10³ IU of recombinant murine IFN-β or 10³ IU of recombinant murine IFN-γ were similar.

### Example 2 Larger Trial of Anti-Tumor Activity of Low Dose Oromucosal Interferon-α in Mice Injected with Friend Erythroleukaemia Cells

In order to confirm the results of Example 1, a larger trial was performed. One hundred fifty 8 week-old female DBA/2 mice were challenged iv with 1 x 10⁵ FLC (2 x 10⁴ FLC LD₅₀) on day 0. Mice were treated with the type and dose of IFN indicatcd, administered by the in/or route in a 10 µl volume twice a day for 10 consecutive days. These were 10 mice in each treatment group. The results are summarized in Table 2.

**Table 2**

| Group | Treatment | Dose | Excipient | Mean day of death ± SE |
|---|---|---|---|---|
| 1 | None | - | None | 9.5 ± 0.2 |
| 2 | Mock Mu IFN-α | - | BSA/PBS | 9.6 ± 0.2 |
| 3 | Mu IFN-α | 10⁴ IU | BSA/PBS | 29.4 ± 25.6 %* |
| 4 | Mu IFN-α | 10³ IU | BSA/PBS | 11.6±0.2 |
| 5 | Mu IFN-α | 10² IU | BSA/PBS | 10.3±0.2 |
| 6 | Hu IFN-α 1-8 | 10⁴ IU | BSA/PBS | 18.2 ±0.8 |
| 7 | Hu IFN-α 1-8 | 10³ IU | BSA/PBS | 13.1 ± 0.8 |
| 8 | HU IFN-α 1-8 | 10² IU | BSA/PBS | 11.4± 0.5 |

| | | | | |
|---|---|---|---|---|
| IU : International reference units | | | | |
| BSA/PBS : 100 µg/ml of BSA in PBS pH 7.4 | | | | |
| Mu IFN-α : natural mixture of murine IFN-α subtypes | | | | |
| Hu IFN-α : single recombinant human IFN-α isotype | | | | |
| * : Some of the animals in this group were still alive at 100 days post treatment | | | | |

IFN-α administered by the in/or route exhibits a marked anti-tumor activity in mice challenged iv with FLC. Indeed, some IFN-treated mice can be considered to be cured, as they were still alive more than 100 days after inoculation of 100,000 FLC, in a system in which 4 to 5 FLC cells are sufficient to kill an animal.

Pure preparations of a single recombinant IFN-α subspecies, IFN-α 1-8, a natural mixture of multiple IFN-α subtypes, Mu IFN-α, recombinant murine IFN-β, and recombinant murine IFN-γ exhibit marked anti-tumor activity in this model when administered by the in/or route, strongly suggesting that the observed anti-tumor activity of oromucosally administered IFN preparations is indeed due to the IFN component of these preparations.

### Example 3 Effect of Route of Administration of Interferon on Anti-Tumor Activity

The effect of in/or administered IFN was compared with that of IFN given by conventional routes. Eight week-old female DBA/2 mice were challenged iv with 1 x 10⁵ FLC (2 x 10⁴ FLC LD₅₀) on day 0. Mice were treated twice a day for 10 consecutive days with 10⁴ IU of Mu IFN-α (the optimal dose as determined in Example 2) administered by the route indicated. There were 6 mice in each treatment group, and in each case the excipient for the Mu IFN-α was BSA in PBS. The results are summarized in Table 3.

**Table 3**

| Group | Treatment | Route | Mean day of death ± SE |
|---|---|---|---|
| 1 | None | | 9.6± 0.2 |
| 2 | Mu IFN-α | Intranasal/oral | 30 ± 25.85 %* |
| 3 | Mu IFN-α | Oral | 18.5 ± 2.0 |
| 4 | Mu IFN-α | Gastric | 13.5 ± 1.3 |
| 5 | Mu IFN-α | Subcutaneous | 23.5 ± 1.6 |
| 6 | Mu IFN-α | Intramuscular | 23.7 ± 1.8 |
| 7 | Mu IFN-α | Intravenous | 25.0 ± 1.2 |
| 8 | Mu IFN-α | Intraperitoneal | 26.7 ± 4.1 |

| | | | |
|---|---|---|---|
| IU : international reference units | | | |
| BSA/PBS : 100 µg/ml of BSA in PBS pH 7.4 | | | |
| * : Some of the animals in this group were still alive 100 days after inoculation of FLC cells. | | | |

The oromucosal (or in/or) route of administration is at least as effective as the commonly used parenteral routes, such as iv, intramuscular (im), and subcutaneous (sc) injection, if not more effective. The in/or route was in fact as effective as ip injection, which is considered to be the most effective route in mice challenged iv with FLC. In contrast, administration of the same dose of IFN directly into the mouth appeared to be less effective than combined intranasal and oral administration, while introduction of IFN directly into the stomach of animals via a tube was considerably less effective.

### Example 4 Effect of Oromucosal Interferon on Expression of Cellular Proteins

IFN-α is known to induce the expression of a number of cellular proteins following binding of the protein to its cell surface receptor. These proteins are thought to provide a useful marker of IFN action.

We evaluated the effect of IFN-α administered via the in/or route on the expression of three IFN-induced proteins, MHC class I antigens, Ly 6A/E antigen and 2'-5'-oligoadenylate synthetase.

Treatment of DBA-2 mice (H-2K^{d}) with up to 20,000 IU of Mu IFN-α by the in/or route did not significantly increase H-2-K^{d} expression on peripheral blood lymphocytes, monocytes or granulocytes under conditions where as little as 20 IU of Mu IFN-α given ip markedly increased the expression of H-2-K^{d} antigens on both peripheral blood monocytes and granulocytes. Indeed, expression on monocytes was slightly suppressed.

Similarly, treatment of mice with up to 20,000 IU of IFN-α via the in/or route had no significant effect on the expression of Ly6 A/E antigens, the expression of which is markedly enhanced on the surface of a variety of lymphoid cells following parenteral treatment with type I IFN (Dumont *et al*; *J*. *Immunol,* 1986137: 201-210). Similar results were obtained with 200 or 20,000 IU of either Mu IFN-α or Hu IFN-α 1-8 via the in/or route.

Treatment of either Swiss or DBA/2 mice with as little as 20 IU of Mu IFN-α injected ip resulted in a marked increase in 2'-5'-oligoadenylate synthetase activity in both peripheral blood mononuclear cells and splenocytes. In contrast, in the same experiment treatment of mice with up to 20,000 IU of Mu IFN-α via the in/or route did not significantly increase the expression of 2'-5'-oligoadenylate synthetase activity. Furthermore, treatment with 200 or 20,000 IU of either Mu IFN-α or Hu IFN-α 1-8 by the in/or route had no significant effect on 2'-5'-oligoadenylate synthetase activity at any of the time points tested up to 10 days after the start of IFN treatment.

### Example 5 Bioavailability of Interferon Following Oromucosal Administration

In order to examine the bioavailability and pharmacokinetics of IFN, mice, which have the most favorable drug-blood volume ratio for such studies, were treated with a single high dose of recombinant IFN-α labeled to the highest specific radioactivity possible with ¹²⁵I.

A pure preparation of 70 x 10⁶ IU of Hu IFN-α 1-8 was taken up in 1.4 mls of PBS, and iodinated as described by Mogensen *et al,* (*Int. J. Cancer,* 1981 **28**: 575-582) using a modification of the chloramine-T method described by Hunter and Greenwood (*Nature*, 1962 **194**: 495-496).

The ¹²⁵I-labeled Hu IFN-α 1-8 (lot no. CGP35269-1) exhibited a biological activity of 2 x 10⁷ IU/ml when assayed on human WISH cells challenged with VSV and 1 x 10⁶ IU/ml when assayed on mouse L929 cells challenged with VSV.

Six to seven week-old female Swiss mice were injected iv, ip, or treated in/or with 2 x 10⁷ IU equivalent to 1 x 10⁶ murine IU of ¹²⁵I Hu IFN-α 1-8 (1.0369 x 10⁷ cpm/mouse). At the time points indicated, three mice per group were sacrificed, blood was collected, and the volume determined. Kidney, liver, lung, spleen, and stomach/esophagus were harvested, blotted, and weighed to a precision of ± 1.0 µg. The radioactivity of each sample was determined individually using a gamma counter. Whole blood was then separated by centrifugation (800 g x 10 min., 4°C), the serum was harvested, counted, and frozen at -80°C. The serum was then assayed for IFN content using a standard bioassay on both human WISH cells and on mouse L929 cells as described above. The radioactive material present in the samples of serum was then isolated by affinity immunoprecipitation and analyzed by SDS-PAGE.

Very high levels of radioactivity (> 2 x 10⁶ cpm/ml) were detected in the peripheral blood of animals 5 min. after injection of 1.0369 x 10⁷ cpm/mouse of ¹²⁵ I-labeled Hu IFN-α 1-8 by iv bolus. The amount of radioactivity present in whole blood then declined progressively at 15 and 30 min. The levels of radioactivity detected in the peripheral blood of animals 5 min. after ip injection of 1.0369 x 10⁷ cpm of ¹²⁵I Hu IFN-α-1-8 were approximately twenty fold lower than the levels detected following an iv bolus. The levels of radioactivity then increased progressively at 15 and 30 min. post-injection. The levels of radioactivity detected in the blood of animals at 5,10 or 15 min. after the in/or administration of ¹²⁵I IFN-α 1-8 were significantly lower than those detected at a given time following ip injection of the same quantity of radiolabelled IFN. For all three routes of administration, higher levels of radioactivity were detected in serum than in whole blood following in/or administration of ¹²⁵I-labeled IFN-α 1-8. The lower levels of radioactivity detected per ml of whole blood compared with the same volume of serum reflect the effectively larger volume of serum counted after removal of the cellular component of whole blood.

Samples of serum from all the mice in the study were assayed for the presence of biologically active IFN using a standard bioassay, as described above, and showed readily detectable levels of biologically active IFN in the serum of all the animals injected either iv or ip with ¹²⁵I Hu IFN-α 1-8 at all the time points tested. In contrast, no biologically active IFN was detected in the serum of any of the animals at any of the time points tested following the in/or administration of IFN, in spite of the presence of relatively high levels of radioactivity in the serum of these animals.

In order to determine whether the radioactive material detected in the serum of animals treated with ¹²⁵I Hu IFN-α 1-8 does indeed represent native IFN, the samples were immunoprecipitated with protein A-G Agarose, in order to precipitate immunoglobulins present in the samples, treated with an affinity-purified polyclonal anti-IFN-αantibody, and further immunoprecipitated. The samples were then subjected to SDS-polyacrylamide gel electrophoresis (SDS-PAGE) as described above.

SDS-PAGE analysis of the radioactive material in serum following iv or ip injection of ¹²⁵I Hu IFN-α 1-8 revealed a single homogenous band migrating with an electrophoretic mobility identical to that of uninjected ¹²⁵I Hu IFN-α 1-8. The apparent molecular weight of the material was estimated to be approximately 20000 Daltons, which corresponds exactly to the molecular weight of native Hu IFN-α 1-8. In contrast, none of the samples of serum from mice treated in/or with ¹²⁵I IFN-α 1-8 contained any material with an apparent molecular weight similar to that of native IFN, even though an identical quantity of radioactive material was loaded on to each gel.

The tissue distribution of radiolabelled material revealed very high levels of radioactivity in the kidneys, high levels in the liver, lung, and spleen of animals 5 min. after the iv injection of ¹²⁵I IFN-α 1-8. The level of radioactivity present in each of these four organs was then found to decrease progressively at 15 and 30 min. In contrast, the level of radioactivity in the stomach increased progressively at 15 and 30 min. to reach a level comparable to that present in the serum of animals 30 min. after an iv bolus.

Administration of ¹²⁵I IFN-α 1-8 by ip injection resulted in peak levels of radioactivity in all the tissues examined within 15 min., followed by a decline at 30 min. Similarly, in/or administration of ¹²⁵I Hu IFN-α 1-8 resulted in peak levels of radioactivity in all the tissues studied after 15 min. with some decline in the levels of radioactivity present at 30 min. The levels of radioactivity present in the stomach/esophagus were an order of magnitude greater than those detected in any other organ following the in/or administration of ¹²⁵I-labeled IFN-α 1-8, and were markedly higher than the levels present in these tissues following parenteral administration of the same quantity of radiolabelled Hu IFN-α 1-8 by either the iv or ip routes.

### Example 6 Pharmacokinetics of Interferon Following Intranasal/Oral Administration

For precise determination of the pharmacokinetics of Hu IFN-α 1-8, mice were treated iv, ip or in/or with 1.0369 x 10⁷ cpm/mouse of ¹²⁵I-labeled Hu IFN-α 1-8, and the levels of radioactivity present in both whole blood and serum were determined at a series of time points over a 24 hour period.

The pharmacokinetic profile of ¹²⁵I-labeled Hu IFN-α 1-8 present in the blood of mice after an iv bolus closely followed a logarithmic clearance curve. This agreed with results of a previous study carried out in mice using a closely related molecule, recombinant human α A/D (Bgl) (Bohoslawed *et al*; *J. IFN Res.,* 1986 6: 207-213). The amount of bioavailable material, calculated from the area under the curve of concentration versus time, was also similar to that for human α A/D. A biphasic time-consuming clearance curve was observed following an iv bolus of ¹²⁵I IFN-α 1-8, which is characteristic of substances which are cleared through the kidneys, in agreement with the results of Example *4*. The pharmacokinetics of ¹²⁵I-labeled IFN-α 1-8 following ip injection closely resembled those previously reported for IFNs administered im.

Readily detectable levels of biologically active IFN were present in the serum of all the animals following either an iv bolus or ip injection of ¹²⁵I-labeled IFN-α 1-8.

The Friend erythroleukaemia model constitutes a very severe preclinical test of anti-tumor activity, since FLC are highly malignant and metastasize to both the liver and spleen when injected iv. Indeed, results obtained using this model were the basis for the adoption of parenteral injection of IFN-α for treatment of human cancers. Thus, in all the experiments carried out in this study all the untreated animals and animals treated with control preparations died within 10 to 11 days. Injection of only 4 or 5 FLC cells will kill a mouse if no treatment is given. In contrast, some of the animals treated with murine IFN-α by the oromucosal route are still alive more than 100 days after inoculation of 10⁵ FLC, and may be considered to be cured.

Indeed, judging from previous work, IFN-α administered by the oromucosal route appears to be more effective than cyclophosphamide, 5-fluorouracil, or methotrexate administered parenterally, which increase survival time by only a few days in animals injected with FLC (Gresser *et al*, *J. Natl. Cancer Inst.,* 1988 **80**: 126-131). Other drugs, such as cisplatin, vincristine, doxorubicin, bleomycin or etoposide are ineffective against this tumor (Gresser *et al*, *J. Natl*. *Cancer Inst*, 1988 **80**: 126-131).

Similarly, IFN-α administered by the oromucosal route appears to be more effective against FLC than other cytokines such as IL-1β, IL-2 and TNF-α administered systemically, which exhibit very little activity in this model.

Previous work has shown that IFN administered parenterally is one of the most active anti-tumor drugs in this model, and that IFN therapy is effective even when initiated after tumor metastases are already present in the liver (Gresser *et al*, *Intl. J. Cancer,* 1987 **39**: 789-792). The present results show that IFN administration by the oromucosal route is equally, or even more, effective.

Daily injections of IFN-α given together with a single dose of cyclophosphamide markedly increased the survival of lymphoma-bearing AKR mice compared to animals treated with either agent alone, when therapy was started after diagnosis of the lymphoma (Gresser *et al, Eur. J. Cancer,* 1978 **14**: 97-99). Successful combination therapy using IFN-αZβ and BCNU, cis-DDP (cisplatin), methotrexate, adriamycin, and α-difluoromethyl ornithine has also been reported in various pre-clinical animal tumor models. Combination therapy with 5-fluorouracil (5-FU) and IFN has also been reported to be of benefit in the treatment of metastatic colon cancer in man (Ernstoff *et al, Journal of Clinical Oncology,* 1989 **7**: 1764-1765). There are, however, other studies which have reported a decreased anti-tumor activity when IFN therapy was combined with the use of cyclophosphamide (Marquet *et al, Int. J. Cancer,* 1983 **31**: 223-226; Lee *et al, Biochem. Pharmacol.,* 1984 **33**: 4339-3443), adriamycin (Blackwill *et al, Cancer Res.,* 1984 **44**: 904-908), or 5-FU (*Marquet et al,* 1985 **109**: 156-158), *i.e.* precisely the same drugs which have been shown to exert a beneficial effect when used in combination with parenteral IFN therapy. Combinations between IFN and other chemotherapy agents can readily be tested using methods described herein.

Combined interleukin-1 (IL-1) and IFN-α/β therapy results in a synergistic anti-tumor effect in mice injected with FLC (Belardelli *et al*, *Int. J. Cancer,* 1991 **49**: 274-278). The same treatment also exerts a marked anti-tumor effect against a metastatic variant (p11-R-Eb) of the Eb lymphoma, against which either agent alone is ineffective (Gabriele *et al*, *Invasion Metastasis,* 1993 **13**: 147-162). Of all the cytokines tested, IL-1 was found to be the most effective when combined with parenteral type I IFN therapy.

Combination therapy with the angiogenesis inhibitor AGM-1470 [(Chloroacetyl)-carbamic acid (3R-(3α, 4α (2R*, 3R*), 5β, 6β))-5-methoxy-4-(2-methyl-3-(3-methoxy-2-butenyl)oxiranyl)-1-oxaspiro(2.5)oct-6-yl ester] given together with IFN-α/β resulted in a markedly increased anti-tumor effect compared to that observed with either agent alone (Brem *et al*, *J. Pediatric Surgery,* 1993 **28**: 1253-1257).

It has been shown that hyperthermia enhances the anti-tumor action of IFN-α/β against the Lewis lung carcinoma (Yerushalmi *et al*, *Proc. Soc. Exp. Biol. Med*., 1982 **169**: 413-415). Arginine butyrate has also been shown to potentiate the anti-tumor action of IFN-α (Chany and Cerutti, *Int. J. Cancer,* 1982 **30**: 489-493).

Comparison of the degree of protection obtained when a given type and dose of IFN was administered by the oromucosal route compared to the results obtained following systemic administration (ip injection) showed that parenteral administration of IFN was in some cases marginally more effective, and in other cases no more effective, than oromucosal administration.

The results of the biomarker pilot study show quite clearly that none of the three biomarkers tested (MHC class I antigen, Ly6 A/E antigen, and 2'-5'-oligoadenylate synthetase activity) adequately reflects the very marked antitumoral activity exhibited by IFN-α administered by the oromucosal route.

The contrast between the very marked increase in the expression of all three IFN-induced proteins observed in all the experiments undertaken following the ip injection of as little as 20 IU of IFN-α and the absence of any detectable effect following the administration of up to 20,000 IU of IFN-α via the oromucosal route is striking.

Although we cannot exclude the possibility that an effect on one or other of the biomarkers would have been observed at an earlier or intermediate time point, this seems to be unlikely, as IFN acts on the transcription of the genes coding for these proteins and thus one would not expect to see an effect on any of these biomarkers until a number of hours after IFN treatment.

Again, although we cannot exclude the possibility that a systemic effect on one of the other numerous IFN-induced proteins would have been observed following treatment with IFN-α by the oromucosal route, this seems unlikely, as this would imply differential regulation of the expression of certain IFN-induced genes. It is entirely possible, however, that an effect on an IFN biomarker may be observed locally, for example, in nasal lymphocytes following administration of IFN-α via the oromucosal route.

In keeping with the absence of a detectable effect on the biomarkers studied, no consistent effect was observed on any of the hematological or blood chemistry parameters monitored during oromucosal IFN therapy, even in animals treated with up to 20,000 IU of IFN-α.

Readily detectable levels of radiolabelled material were found in both whole blood and serum of animals following oromucosal administration of ¹²⁵I-labeled IFN-α 1-8. These results contrast with the results of previous studies, which failed to detect IFN in the serum of animals even after the oral administration of large quantities of unlabelled IFN. However, the radioactive material detected in both whole blood and serum following oromucosal administration was biologically inactive. Furthermore, the results of SDS-PAGE analysis showed that this material was of low molecular weight, and most probably reflected the absorption of degradation products following digestion of IFN in the stomach and small intestine. Analysis of the tissue distribution of radiolabelled material following oromucosal administration revealed markedly higher levels of radioactivity in the stomach than in any of the other organs tested. Our results show quite clearly that even though biologically active IFN was not absorbed following oromucosal administration, this treatment does nevertheless exert a statistically significant antitumor activity *in vivo.*

Without wishing to be bound by any proposed mechanism for the observed beneficial effect, our results suggest that oromucosally administered IFN exerts its effects against cancer cells via a presently undefined novel mechanism, which does not involve a direct action of exogenously administered IFN, or the induction of endogenous IFN. This is supported by the absence of detectable levels of circulatory IFN or of the three biomarkers tested. It appears that this mechanism may act at least partly by stimulation of the abundant lymphoid tissue surrounding the nasopharyngeal and oral cavities. Since we have shown that oromucosal IFN is at least comparable in efficacy to systemically administered IFN, our results provide strong support for administration of IFN by the oromucosal route in the treatment of cancer. This could have important implications for the clinical use of IFN.

## Claims

1. Use of human interferon in the manufacture of a medicament for the treatment of cancer in a human by administration by contact with the mucosal lining of the mouth and throat of the recipient human of from 10⁴ IU to 20 x 10⁶ IU of interferon per day.

2. The use according to claim 1 wherein said cancer is multiple myeloma, hairy cell leukemia, chronic myelogenous leukemia, low grade lymphoma, cutaneous T-cell lymphoma, carcinoid tumor, cervical cancer, sarcomas including Kaposi's sarcoma, kidney tumor, carcinomas including renal cell carcinoma, hepatic cellular carcinoma, nasopharyngeal carcinoma, hematological malignancies, colorectal cancer, glioblastoma, laryngeal papilloma, lung cancer, colon cancer, malignant melanoma, or brain tumors including malignant brain tumor.

3. The use according to claim 1 or 2 in which the medicament comprises a single dose of the interferon.

4. The use according to claim 1 or 2 in which the medicament comprises a plurality of smaller doses of the interferon sufficient to elicit a response equivalent to that of a single dose, without inducing a pathological response.

5. The use according to any of claims 1-4 in which the medicament provides for continuous administration of a dose of interferon over a period of time sufficient to elicit an antineoplastic response equivalent to that of a single dose, without inducing a pathological response.

6. The use according to any one of claims 1-5 in which the medicament comprises another cytokine or an interferon-inducer.

7. The use according to any one of claims 1 to 6 in which the medicament is adapted to administer from 1 x 10⁴ to 1 x 10⁶ IU of interferon per day.

8. The use according to any one of claims 1 to 7 in which the medicament includes another therapeutic agent for simultaneous, separate or sequential therapy.

9. The use according to claim 8 in which the therapeutic agent is selected from the group consisting of cytostatic, anticancer, and antiangiogenic agents.

10. The use according to any one of claims 1 to 9 in which the interferon is a Type I interferon.

11. The use according to claim 10 in which the interferon is selected from the group consisting of IFN-α, IFN-β, IFN-ω, consensus IFN, and mixtures thereof.

12. The use according to claim 11 in which the Type I interferon is IFN-α.

13. The use according to any one of claims 1-9 in which the interferon is a Type II interferon.

14. The use according to claim 13 in which the Type II interferon is IFN-γ.

15. The use according to claim 10 or 13 in which the interferon is recombinant material.

## Patentansprüche

1. Verwendung von menschlichem Interferon zur Herstellung eines Medikaments zur Behandlung von Krebs bei einem Menschen durch Verabreichung mittels Kontakt mit der Schleimhautauskleidung des Mund- und Rachenbereichs eines menschlichen Rezipienten von 104 IE bis 20 x 10⁶ IE Interferon pro Tag.

2. Verwendung nach Anspruch 1, worin der Krebs multiples Myelom, Haarzellenleukämie, chronische myelogene Leukämie, minderschweres Lymphom, kutanes T-Zell-Lymphom, karzinoider Tumor, Zervixkarzinom, Sarkom einschließlich Kaposi-Sarkom, Nierentumor, Karzinom einschließlich Nierenzellkarzinom, Leberzellenkarzinom, Nasopharynxkarzinom, hämatologische Malignität, Kolorektalkarzinom, Glioblastom, Larynxpapillom, Lungenkrebs, Kolonkrebs, malignes Melanom oder Himtumor einschließlich maligner Himtumor ist.

3. Verwendung nach Anspruch 1 oder 2, worin das Medikament eine einzelne Dosis des Interferons umfasst.

4. Verwendung nach Anspruch 1 oder 2, worin das Medikament zahlreiche kleine Dosen des Interferons umfasst, die ausreichen, um eine Reaktion hervorzurufen, die jener einer einzelnen Dosis entspricht, ohne eine pathologische Reaktion zu induzieren.

5. Verwendung nach einem der Ansprüche 1 bis 4, worin das Medikament für kontinuierliche Verabreichung einer Dosis von Interferon über eine ausreichende Zeitspanne hinweg sorgt, um eine antineoplastische Reaktion hervorzurufen, die jener auf eine einzelne Dosis entspricht, ohne eine pathologische Reaktion zu induzieren.

6. Verwendung nach einem der Ansprüche 1 bis 5, worin das Medikament ein weiteres Cytokin oder einen Interferon-Induktor umfasst.

7. Verwendung nach einem der Ansprüche 1 bis 6, worin das Medikament an eine Verabreichung von 1 x 10⁴ bis 1 x 10⁶ IE Interferon pro Tag angepasst ist.

8. Verwendung nach einem der Ansprüche 1 bis 7, worin das Medikament ein weiteres Therapeutikum zur gleichzeitigen, getrennten oder darauffolgenden Therapie umfasst.

9. Verwendung nach Anspruch 8, worin das Therapeutikum aus der aus zytostatischen, Antikrebs- und antiangiogenen Mitteln bestehenden Gruppe ausgewählt ist.

10. Verwendung nach einem der Ansprüche 1 bis 9, worin das Interferon ein Interferon vom Typ I ist.

11. Verwendung nach Anspruch 10, worin das Interferon aus der aus IFN-α, IFN-β, IFN-ω, Consensus-IFN und Gemischen davon bestehenden Gruppe ausgewählt ist.

12. Verwendung nach Anspruch 11, worin das Interferon vom Typ IIFN-α ist.

13. Verwendung nach einem der Ansprüche 1 bis 9, worin das Interferon ein Interferon vom Typ II ist.

14. Verwendung nach Anspruch 13, worin das Interferon vom Typ II IFN-γ ist.

15. Verwendung nach Anspruch 10 oder 13, worin das Interferon rekombinantes Material ist.

## Revendications

1. Utilisation de l'interféron humain dans la fabrication d'un médicament pour le traitement du cancer chez un humain par administration par contact , avec le revêtement mucoseux de la bouche et de la gorge de l'humain récepteur de 10⁴ UI à 20 x 10⁶ UI d'interféron par jour.

2. Utilisation selon la revendication 1 où ledit cancer est un myélome multiple, une leucémie des cellules velues, une leucémie myélogène chronique, un lymphome de faible degré, un lymphome des cellules T cutanées, une tumeur carcinoïde, un cancer cervical, des sarcomes comprenant les arcome de Kaposi, une tumeur au rein, des carcinomes comprenant un carcinome des cellules rénales, un carcinome cellulaire hépatique, un carcinome naso-pharyngé, des malignités hématologiques, un cancer colorectal, un gliobastome, un papillome laryngé, un cancer des poumons, un cancer du côlon, un mélanome malin ou des tumeurs au cerveau comprenant des tumeurs malignes.

3. Utilisation selon la revendication 1 ou 2 dans laquelle le médicament comprend une seule dose de l'interféron.

4. Utilisation selon la revendication 1 ou 2 dans laquelle le médicament comprend un certain nombre de plus petites doses de l'interféron, suffisantes pour éliciter une réponse équivalente à celle d'une seule dose sans induire une réponse pathologique.

5. Utilisation selon l'une quelconque des revendications 1 -4 dans laquelle le médicament permet l'administration continue d'une dose d'interféron sur une période de temps suffisante pour éliciter une réponse anti-néoplasique équivalente à celle d'une seule dose, sans induire de réponse pathologique.

6. Utilisation selon l'une quelconque des revendications 1-5 dans laquelle le médicament comprend une autre cytokine ou un inducteur d'interféron.

7. Utilisation selon l'une quelconque des revendications 1 à 6 dans laquelle le médicament est adapté à administrer de 1 x 10⁴ à 1 x 10⁶ UI d'interféron par jour.

8. Utilisation selon l'une quelconque des revendications 1 à 7, dans laquelle le médicament comprend un autre agent thérapeutique pour thérapie simultanée, séparée ou séquentielle.

9. Utilisation selon la revendication 8 dans laquelle l'agent thérapeutique est sélectionné dans le groupe consistant en agent s cytostatique s, anticancéreux, et antiangiogéniques.

10. Utilisation selon l'une quelconque des revendications 1 à 9, dans laquelle l'interféron est un interféron du Type I.

11. Utilisation selon la revendication 10, dans laquelle l'interféron est sélectionné dans le groupe consistant en IFN-α, IFN-β, IFN-ω, IFN en consensus et leurs mélanges.

12. Utilisation selon la revendication 11 dans laquelle l'interféron Type I est IFN-α.

13. Utilisation selon l'une quelconque des revendications 1 -9 dans laquelle l'interféron est un interféron Type II.

14. Utilisation selon la revendication 13 dans laquelle l'interféron Type II est IFN-γ.

15. Utilisation selon la revendication 10 ou 13 dans laquelle l'interféron est une matière recombinante.
